(19) Europäisches Patentamt
European Patent Office
Office européen des brevets

(11) **EP 3 915 640 A1**

(12) **EUROPEAN PATENT APPLICATION**
published in accordance with Art. 153(4) EPC

(43) Date of publication:
**01.12.2021 Bulletin 2021/48**

(21) Application number: **20745606.2**

(22) Date of filing: **19.03.2020**

(51) Int Cl.:
*A61P 31/04* (2006.01)     *A61P 35/00* (2006.01)
*C12N 5/0783* (2010.01)     *A61K 35/17* (2015.01)

(86) International application number:
**PCT/IB2020/052499**

(87) International publication number:
**WO 2020/152661 (30.07.2020 Gazette 2020/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.01.2019  JP 2019007868
04.03.2020  US 202016808989**

(71) Applicant: **GAIA BioMedicine Inc.
Chuo-ku
Fukuoka-shi
Fukuoka 810-0023 (JP)**

(72) Inventors:
• **YONEMITSU, Yoshikazu**
  **Fukuoka-shi, Fukuoka 819-0395 (JP)**
• **HARADA, Yui**
  **Fukuoka-shi, Fukuoka 819-0395 (JP)**

(74) Representative: **Vossius & Partner
Patentanwälte Rechtsanwälte mbB
Siebertstraße 3
81675 München (DE)**

Remarks:
The restoration of the right of priority granted by the receiving Office is effective before the EPO (R. 49ter.1(a) PCT).

(54) **PRODUCTION METHOD FOR CELL POPULATION INCLUDING NK CELLS**

(57) An object of the present invention is to provide an effective method for producing a population of NK cells for cell therapy. Another object of the present invention is to improve in vitro amplification efficiency of NK cells. A still another object of the present invention is to flexibly increase signals required for licensing of NK cells. There is provided a method for producing a cell population including NK cells, which comprises preparing a cell population of mononuclear cells originating in a plurality of donors and including NK cells, and incubating the prepared population of mononuclear cells under conditions effective for treating and proliferating NK cells to proliferate NK cells.

**EP 3 915 640 A1**

**Description**

Technical Field

The present invention relates to a method for producing a cell population including natural killer cells (NK cells) and use thereof.

Background Art

Malignant tumors are leading causes of death of Japanese people, and establishing measures to counter them is considered to be urgent necessity. In particular, development of novel therapeutic techniques for intractable malignant tumors in advanced stages, which are resistant to existing therapies consisting of surgical operations, radiotherapies and chemotherapies, is very important and meaningful. In recent years, immunotherapies such as therapies utilizing an immunity checkpoint inhibitor or chimeric antigen receptor (CAR) gene-modified T cell (CAR-T therapy) attract attention as the fourth treatment methods. However, since many of them use T cells that are activated when they recognize an antigen as an effecter, they suffer from a fundamental obstacle of limitation to a specific antigen.

As immunotherapies using NK cells that act as a major factor of natural immunity, NK cell therapies by proliferating NK cells in vitro, and then administering them to a patient attract attention as therapies causing comparatively few side reactions. However, number of NK cells that can be obtained from peripheral blood or the like is comparatively small, and in addition, the in vitro proliferation rate thereof is low. Therefore, techniques for culturing NK cells to proliferate them have been examined. For example, Patent document 1 proposes a method for amplifying NK cells, which comprises the step of preparing a cell population including NK cells, the step of eliminating T cells from the cell population including NK cells, and the step of culturing the cells remained after the removal in a medium containing only IL-2 as cytokine without using feeder cells, and a pharmaceutical composition for cell therapy, which comprises a cell population including NK cells obtained by the amplification. Patent document 2 proposes a method for preparing NK cells, which comprises the step of amplifying hematopoietic precursor cells under a single type culture conditions using IL-15, SCF, IL-7, and Flt3L, and the step of deriving the cells obtained in the amplification step into NK cells by differentiation under culture conditions using IL-2 in 5, 6, 7, 8, or 9 days, and a pharmaceutical composition for cell therapy comprising a cell population including the prepared NK cells.

The inventors of the present invention reported a CD16-positive, CD56-highly expressing, CD57-negative, NKG2C-positive, NKG2A-negative to lowly expressing, and CD94-positive NK cell and a cell population including the NK cell (Patent document 3), and also reported a CD3-negative cell, which expresses a chemokine receptor and a cell adhesion molecule (Patent document 4).

On the cell surfaces of NK cells, receptors that recognize HLA class I molecules are expressed. Among these receptors, those of KIR (Killer cell Immunoglobulin-like Receptor) family show diversity like HLA. In hematopoietic stem cell transplantation, it is recently considered that not matching HLA as much as possible, but use of intentional KIR/HLA mismatch between donor and recipient, such as selecting donor so that recipient should not have any ligand to KIR of donor NK cells, can provide more desirable results from the aspects of recurrence, GVHD (graft versus host disease), and prognosis, and the latter has come to be effectively used. However, as for the antitumor activity of NK cells, the report of examination in which NK cells were cultured with feeder cells that matched or mismatched with an inhibitory KIR ligand (in the mismatching cells, one or more ligands existing in the NK cell donor are missing), and how much the HLA class I-KIR interactions would influence human NK cell proliferation in an allogeneic environment was examined (Non-patent document 1) suggests that, when increase in activity of NK cells as for antitumor effects is desired, less signals from KIR are more favorable. Further, the recent report concerning allogenic response characteristics of HLA-hetero NK cells (in the case of one-way match) against a tissue derived from HLA haplotype homozygous (HLA-homo) iPS cells (Non-patent document 2) strongly suggests that mixed culture including HLA/KIR mismatch cannot be realized for culture of NK cells.

Prior Art References

Patent documents

Patent document 1: Japanese Patent Unexamined Publication (KOKAI) No. 2013-27385 (Japanese Patent Nos. 5572863 and 5989016)
Patent document 2: Japanese Patent Unexamined Publication (KOKAI) No. 2014-226079 (Japanese Patent Nos. 5511039 and 6164650)

Patent document 3: Japanese Patent Unexamined Publication (KOKAI) No. 2018-193303
Patent document 4: Japanese Patent Application No. 2018-059624 (not published yet at the time of the filing of the present application)

Non-patent documents

Non-patent document 1: Mingus J.J. Rose et al., Killer Ig-Like Receptor Ligand Mismatch Directs NK Cell Expansion In Vitro, The Journal of Immunology, 2009, 183:4502-4508

Non-patent document 2: Hiroshi Ichise et al., NK Cell Alloreactivity against KIR-Ligand-Mismatched HLA-Haploidentical Tissue Derived from HLA Haplotype-Homozygous iPSCs, Stem Cell Reports, 2017, 9:853-867

Disclosure of the Invention

Object to be Achieved by the Invention

The inventors of the present invention are developing cell populations including highly active NK cells. However, since amounts of peripheral blood and blood obtained by the apheresis method used as the raw materials are limited, NK cell populations for therapeutic treatment cannot be stored in an off-the-shelf state for the treatments in future.
By the way, according to the investigations by the inventors of the present invention, in vitro amplification efficiencies of NK cells of some donors may be extremely bad.
Further, it is considered that, outside the body, immature NK cells that have experienced binding with the ligand (HLA class I) are "licensed" in the process of the differentiation from hematopoietic stem cells in the bone marrow get an ability for "missing-self response" for detecting cells of which expression of self-HLA class I is reduced after the maturation, but the signal required for the licensing of NK cells differentiated from iPS cells or embryonic stem (ES) cells and matured may be insufficient for acquiring antitumor effect.

Means for Achieving the Object

The present invention provides the followings.

[1] A method for producing a cell population including NK cells, which comprises preparing a cell population of mononuclear cells originating in a plurality of donors and including NK cells, and incubating the prepared population of mononuclear cells under conditions effective for treating NK cells.
[2] The production method according to 1, wherein the step of preparing a population of mononuclear cells comprises the step of removing CD3-positive cells.
[3] The production method according to 1 or 2, wherein the step of preparing a population of mononuclear cells comprises the step of removing CD34-positive cells.
[4] The production method according to any one of 1 to 3, wherein the step of preparing a population of mononuclear cells comprises the step of obtaining a population of mononuclear cells from peripheral blood collected from a plurality of donors.
[5] The production method according to any one of 1 to 4, wherein the step of preparing a population of mononuclear cells comprises the step of obtaining a population of mononuclear cells from apheresis blood collected from a plurality of donors.
[6] The production method according to any one of 1 to 3, wherein the step of preparing a population of mononuclear cells consists of preparing a population of mononuclear cells derived from any one selected from the group consisting of embryonic stem (ES) cells, induced pluripotent stem (iPS) cells, and adult stem cells originating in a plurality of donors.
[7] The production method according to any one of 1 to 6, wherein the plurality of donors include one donor and another donor having a genotype of at least one of HLA or KIR different from that of the foregoing one donor.
[8] A cell population including NK cells, which has the following characteristics:

    (1) the cell population originates in a plurality of donors,
    (2) the cell population shows a cytotoxic activity of 50% or higher in co-culture of NK cells as effecter cells (E) and K562 cells as target cells (T) at a mixing ratio of 1:1 (E:T).

[9] A pharmaceutical composition for cell therapy, which comprises a cell population produced by the production method according to any one of 1 to 7.

[10] A pharmaceutical composition for cell therapy, which comprises the cell population according to 8.

[11] The pharmaceutical composition according to 9 or 10, which is for treating an infectious disease and/or cancer.

Effect of the Invention

As the raw material of the cell population, blood or PBMCs of a plurality of persons can be mixed, therefore the amount of the raw material can be increased, and it is expected that the cell population can be prepared in an off-the-shelf state.

NK cells can be stably proliferated independently from donors, and the proliferation ratio thereof can be improved.

It becomes possible to respond to diversification of HLA-KIR matching, and stable activation can be expected.

It becomes possible to flexibly increase the signals required for licensing of NK cells, and improvement of antitumor effect of an NK cell population derived from iPS cells or embryonic stem cells can be expected.

Brief Description of the Drawings

[Fig. 1] Culture test: From PBMCs of a plurality of persons, CD3-positive cells were removed, respectively, and the cells were cultured for 14 days by using the KBM-501 medium.

[Fig. 2] Summary (left) and statistical analysis (right) of experimental results: When mixed culture was performed, the proliferation ratio was significantly improved compared with culture of the cells derived from a single donor.

[Fig. 3] Tumor cell cytotoxic activity test: Cytotoxic activities of NK cells produced by mixed culture and culture of those derived from a single donor were evaluated on the basis of the cytotoxicity against SKOV3 (human ovarian cancer cell strain) cells as the index.

[Fig. 4-1] Culture in 500-$cm^2$ culture bag: After 30 minutes from the start of the culture and on the day 9 of the culture, the bag was turned over.

[Fig. 4-2] Culture test: Frozen apheresis blood (obtained from two donors) was used as the material. CD3-Positive cells and CD34-positive cells were removed by using an automatic closed system cell processor, and the cells were cultured for 14 days by using a T75 flask or 500-$cm^2$ culture bag, and the KBM-501 medium.

[Fig. 5] Monocyte/NK cell swapping experiment: When the cells were under conditions that signals were available from one or both of KIR3DL1 and KIR3DS1, a population of CD16[high] cells increased. The population of CD16[high] cells can expected to have higher ADCC activity.

Modes for Carrying out the Invention

The present invention relates to a cell population including NK cells.

[Production of cell population]

The cell population of the present invention including NK cells can be prepared by a method comprising the following steps:

preparing a cell population of mononuclear cells originating in a plurality of donors and including NK cells, and incubating the prepared population of mononuclear cells under conditions effective for treating NK cells.

(Preparation of population of mononuclear cells originating in plurality of donors)

The cell population of the present invention is obtained from a population of mononuclear cells originating in a plurality of donors. The population of mononuclear cells mentioned here includes NK cells, and may include mononuclear cells other than NK cells, for example, monocytes. The NK cells may be primary NK cells (NK cells extracted from a living body and have not been subcultured).

The population of mononuclear cells originating in a plurality of donors and including NK cells may be a mixture of a population of mononuclear cells obtained from one donor (it includes NK cells, and may include monocytes) and a population of mononuclear cells obtained from another donor (it includes NK cells, and may include monocytes), or may be a mixture of NK cells obtained from one donor and monocytes obtained from another donor.

As for donor, plurality means two or more, and number of plurality is not particularly limited so long as the aforementioned conditions are satisfied, and for example, a mixture of nuclear cells originating in 3 or more, 4 or more, 7 or more, or 9 or more of donors can be used. The plurality of donors may include the patient himself or herself to whom the cell population including NK cells is to be administered, and a close relative of the patient.

The plurality of donors can be chosen so that they include one donor and another donor whose genotype of at least one of HLA and KIR1 is different from that of the foregoing one donor. KIR includes KIR2DL1, KIR2DL2, KIR2DL3, KIR3DL1, KIR3DL2, KIR2DL4, KIR2DS1, KIR2DS2, KIR2DS3, KIR2DS4, KIR2DS5, KIR3DS1, KIR3DL3, KIR2DL5A, and KIR2DL5B.

As for NK cells, the term license (licensing) means a process of acquiring the ability for "missing-self response (detection of lack of self-HLA)" peculiar to NK cells in the course of differentiation and maturation of the cells. It is considered that, as for human and mouse, only immature NK cells that have experienced bindings with KIR or NKG2A and an HLA class I molecule are licensed in the process of the differentiation of the NK cells from hematopoietic stem cells in the bone marrow to acquire an ability for detecting cells of which expression of self-HLA class I is reduced after the maturation. Examples of KIR known to be involved in the licensing are KIR2DL1, KIR2DL2, KIR2DL3, KIR3DL1, and NKG2A/CD94. The HLA types which NK cells can recognize are all the HLA-C allotypes, about 1/3 of the HLA-B allotypes, a part of the HLA-A allotypes (those having Bw4 motif) among the classical HLAs, as well as HLA-E and HLA-G as non-classical HLAs. According to the present invention, when the plurality of donors are chosen so that they include one donor and another donor having a genotype of at least one of HLA and KIR different from that of the foregoing one donor, KIR and HLA involved in such licensing as described above can be especially taken into consideration.

In one of the preferred embodiments of the present invention, the donors having different genotypes can be chosen so that the repertory of the molecules used for licensing of NK cells should be increased. They may also be chosen so that the inhibitory KIR of NK cells should not be stimulated or should be stimulated.

When the antibody-dependent cellular cytotoxicity (ADCC) activity of NK cells is desired, the donors may be selected so that when a population of mixed mononuclear cells is cultured, CD16high NK cells should increase. As one of the mechanisms of the cytotoxicity of NK cells, ADCC is known, and NK cells bind with antibodies bound to target cells via CD16, which is an Fc receptor on the cell surfaces of NK cells, to injure the target cells. Therefore, it can be expected that increase of CD16high NK cells provides higher ADCC activity. Examples of the way for selection of such combination of donors is that the donors are selected so that NK cells are licensed with a signal or signals from one or both of KIR3DL1 and KIR3DS1 by mixing NK cells.

The mixing is performed before administration of NK cells to an object. The mixing can be performed in various stages, so long as the objective effect of the mixing can be obtained. For example, after bloods obtained from a plurality of donors are mixed, a population of mononuclear cells may be obtained from the mixture, or after populations of mononuclear cells are obtained from blood collected from each donor, and the populations may be mixed. Further, after populations obtained from a plurality of donors are individually cultured, the populations may be mixed. From the aspect of obtaining favorable proliferation, the mixing is preferably performed before culture.

The mixing ratio of the populations of mononuclear cells originating in a plurality of donors can be arbitrarily determined. For example, the cells derived from a plurality of donors may be contained at substantially equal ratios, or cells derived from a specific donor may be contained at a larger or smaller ratio. When NK cells of one donor and monocytes derived from another donor are mixed, the mixing ratio of them may also be arbitrarily determined. For example, NK cells can be mixed with monocytes in 1 to 9 times amount of the amount of NK cells (NK cells:monocytes = 1:1 to 9), or NK cells can be mixed with monocytes in 2 to 4 times amount of the amount of NK cells (NK cells:monocytes = 1:2 to 4). In the explanation of the present invention, mixing ratios of the cells are indicated on the number basis, unless especially indicated.

As the raw material from which the population of mononuclear cells is obtained, blood cells extracted from peripheral blood, cord blood, bone marrow, and/or lymph gland can be used. One class of the preferred raw materials is peripheral blood, and peripheral blood may be collected by the apheresis method. That is, in a preferred embodiment of the present invention, the step of preparing a population of mononuclear cells comprises the step of obtaining a population of mononuclear cells from peripheral bloods collected from a plurality of donors. In another preferred embodiment, the step for preparing a population of mononuclear cells comprises the step of obtaining a population of mononuclear cells from apheresis bloods collected from a plurality of donors.

Such a population of mononuclear cells including NK cells and monocytes can be prepared by using any one of various methods known to those skilled in the art. For example, from blood such as peripheral blood and cord blood, erythrocytes can be removed and monocyte cell fraction can be collected by performing density gradient centrifugation separation under room temperature conditions. Mononuclear cells separated from peripheral blood (peripheral blood mononuclear cells, PBMCs) contain various lymphocytes such as T cells, B cells, NK cells, monocytes, and dendritic cells. In the context of the present invention, CD3-negative and CD56-positive mononuclear cells can be considered to be NK cells. NK cells can be prepared by using any of various methods known to those skilled in the art. For example, methods for separating NK cells from whole blood or PBMCs by removing unnecessary cells are known. Monocytes can be prepared by using any of various methods known to those skilled in the art. For example, methods for separating monocytes from whole blood or PBMCs by removing unnecessary cells are known.

(Removal of CD3-positive cells and removal of CD34-positive cells)

The population of mononuclear cells may contain NK cell precursors, T cells, NKT cell, hematopoietic precursor cells, and so forth in addition to monocytes and NK cells. After amplification, desired NK cells may be chosen by using, for example, specific gravity centrifugation, immunomagnetic beads, FACS, flow cytometry, or the like. For example, NK cells may be selectively separated from cell population by using anti-CD3 antibody, anti-CD 16 antibody, anti-CD34 antibody, anti-CD56 antibody, anti-CD69 antibody, anti-CD94 antibody, anti-CD107a antibody, anti-KIR3DL1 antibody, anti-KIR3DL2 antibody, anti-KIR2DL3 antibody, anti-KIR2DL1 antibody, anti-KIR2DS1 antibody, anti-KIR2DL5 antibody, anti-NKp46 antibody, anti-NKp30 antibody, anti-NKG2D antibody, or the like. The antibody may be a monoclonal antibody, a polyclonal antibody, or the like. The selection of NK cells may be performed by selectively removing T cells, NKT cells, hematopoietic precursor cells, and other cells.

The step of preparing a population of mononuclear cells may include the step of removing T cells, and this step may be carried out by removing CD3-positive cells. The step of preparing a population of mononuclear cells may include the step of removing hematopoietic precursor cells, and this step may be carried out by removing CD34-positive cells. That is, the step of preparing a population of mononuclear cells may include the step of removing CD3-positive cells, and may include the step of removing CD34-positive cells.

The removal of CD3-positive cells and the removal of CD34-positive cells can be performed by separating and removing cells expressing cell surface antigens CD3 and/or CD34 by using immunomagnetic beads such as Dynabeads produced by Dynal Biotech and sold by Invitrogen, and CliniMACS of Miltenyi Biotec. This step is preferably performed so that the NK cells should not be exhausted, and monocytes should not exhibit excessive phagocytic function. Specifically, preferred examples of such an operation include use of magnetic beads having a comparatively small size (unreacted beads not binding with cells can be removed together with supernatant after centrifugation), use of comparatively short time for incubation of beads and the cell population at a low temperature, removing unreacted cells and then obtaining cells not binding to beads as the objective cell population using a column, and so forth. The removal of the cells may also be performed by using an automatic closed system cell processor.

(Use of iPS cells etc.)

The population of mononuclear cells may be one prepared from hematopoietic stem cells derived from any one of embryonic stem (ES) cells, induced pluripotent stem (iPS) cells and adult stem cells. There are known methods for deriving mononuclear cells including NK cells from these stem cells, and those skilled in the art can apply them to the present invention (Domogala A. et al., Natural killer cell immunotherapy: from bench to bedside, Frontiers in Immunology, 2015, 6, doi:10.3389/fimmu.2015.00264; and Zeng J. et al., Generation of "Off-the-Shelf' Natural Killer Cells from Peripheral Blood Cell-Derived Induced Pluripotent Stem Cells, Stem Cell Reports, 2017:9:1796-1812). The stem cells may be modified so that highly active NK cells can be obtained. For example, high affinity CD16 (158V, 158 amino acid is valine) is known, and such a finding can be applied.

(Culture of population of mononuclear cells)

The prepared population of mononuclear cells as a mixture of cells derived from a plurality of donors is incubated under conditions effective for treating NK cells. The incubation may or may not proliferate the cells. In one of the preferred embodiments of the present invention, incubation provides proliferation. The expression of to perform incubation that provides proliferation can be read as to culture or proliferate.

The conditions effective for treating NK cells are conditions suitable for the mixed cells derived from a plurality of donors to exhibit the effect of the mixing. So long as the objective effect is manifested, the medium or isotonic solution, time, temperature, environment, etc. used for suspending the cells are not particularly limited.

The conditions effective for treating NK cells include conditions effective for activation of NK cells. Such conditions may be conditions that the cells are incubated in the presence of factors required for activation, such as IL-2 and IL-15, at an appropriate concentration at 37°C and 5% $CO_2$ for 4 to 18 hours in a saturated steam atmosphere.

The conditions effective for treating NK cells include conditions effective for proliferation of NK cells. The conditions effective for proliferation of NK cells include use of a medium suitable for proliferation of NK cells. Examples of such a medium include the KBM501 medium (Kohjin-bio), CellGro SCGM medium (Cellgenics, Iwai Chemical Co.), X-VIVO15 medium (Lonza, Takara Bio), IMDM, MEM, DMEM, RPMI 1640, and so forth, but not limited to these.

Interleukin 2 (IL-2) may be added to the medium at such a concentration that the object of the present invention can be achieved. The concentration of IL-2 may exceed 2000 IU/mL, and may be 2500 to 2813 IU/mL. It is preferred that IL-2 has the human IL-2 amino acid sequence, and it is preferably produced by a recombinant DNA technique for the safety sake. The concentration of IL-2 may be represented with the Japan reference unit (JRU) or international unit (IU). Since 1 IU is about 0.622 JRU, 1750 JRU/mL is about 2813 IU/mL.

The medium may contain autoserum of test subject, human type AB serum available from BioWhittaker etc., or donated blood human serum albumin available from the Japanese Red Cross Society. The autoserum and human type AB serum are preferably added at a concentration of 1 to 10%, and the donated blood human serum albumin is preferably added at a concentration of 1 to 10%. The test subject may be a healthy subject or a patient suffering from a disease. The medium may contain a composition formulated for proliferation of immunocytes instead of or together with the serum. Such a composition is marketed. For example, those of UltraGro series (AventaCell), and CTS Immune Cell SR (Thermo Fisher Scientific) can be used also for the present invention.

The medium may contain an appropriate protein, cytokine, antibody, compound, and other ingredients on condition that the effect of amplification of NK cells is not impaired. The cytokine may be interleukin 3 (IL-3), interleukin 7 (IL-7), interleukin 12 (IL-12), interleukin 15 (IL-15), interleukin 21 (IL-21), stem cell factor (SCF), and/or FMS-like tyrosine kinase 3 ligand (Flt3L). It is preferred that IL-3, IL-7, IL-12, IL-15, IL-21, SCF, and Flt3L have human amino acid sequences of them, and they are preferably produced by a recombinant DNA technique for the safety sake.

The medium may be exchanged at any time after the start of the culture on condition that a desired cell number of NK cells can be obtained, but it is preferably performed every 3 to 5 days.

The culture vessel may be a commercially available dish, flask, plate, or multi-well plate, but it is not limited to these. In order to obtain the cell population including NK cells for treatment, it is preferable to use a culture vessel that enables culture of many cells and easy handling. Examples of such a culture vessel include a cell culture bag consisting of a material having a high gas permeability.

When a bag is used, it is preferably used with inverting the upper and lower sides thereof during the culture period. During the culture, both NK cells and monocytes may adhere to culture surface. In the case of adhesive cells, the cell density per unit volume as well as cell density per unit area significantly influence the culture efficiency including survival rate and proliferation rate of the cells. By inverting the bag during the culture period, a part of the NK cells and monocytes can be moved to a surface to which comparatively few cells adhere since it was on the upper side, and therefore the culture can be thereby more efficiently performed.

The culture conditions are not particularly limited on condition that the amplification effect of NK cells is not impaired, but culture conditions consisting of 37°C, 5% $CO_2$, and saturated steam atmosphere are common. Since one of the objects of the present invention is to produce NK cells in a large amount, a longer time of culture in the medium that provides more NK cells is more advantageous. The culture period is not particularly limited so long as NK cells can be amplified to a desired cell number. For example, the culture can be performed for 7 to 28 days, 10 to 18 days, or 12 to 16 days, for example, 14 days (Saito S. et al., Ex vivo generation of highly purified and activated natural killer cells from human peripheral blood, Hum. Gene Ther. Methods, 2013;24(4):241-252; and Patent document 1 mentioned above).

The culture may not be performed immediately after the population of mononuclear cells is prepared. The prepared cell population including mononuclear cells may be cryopreserved, then thawed according to the time for administration to a patient, and used for culture of the NK cells. The population of mononuclear cells may be frozen during amplification by the method for amplifying NK cells of the present invention or after completion of the amplification, then thawed according to the time for transplantation to a patient, and used for the transplantation. The freezing and thawing may be performed by using any of methods well known to those skilled in the art. For freezing the cells, any of marketed cell cryopreservation solutions may be used.

The predetermined population of NK cells obtained by culture may contain, in addition to the objective NK cells, NK cell precursors, T cells, NKT cells, hematopoietic precursor cells, etc. After the culture, the objective NK cells or a population thereof may be selected by using, for example, specific gravity centrifugation, immunomagnetic beads, FACS, flow cytometry, or the like. For example, the objective NK cells or a population thereof may be selectively separated by using anti-CD3 antibody, anti-CD16 antibody, anti-CD34 antibody, anti-CD56 antibody, anti-CD69 antibody, anti-CD94 antibody, anti-CD107a antibody, anti-KIR3DL1 antibody, anti-KIR3DL2 antibody, anti-KIR2DL3 antibody, anti-KIR2DL1 antibody, anti-KIR2DS1 antibody, anti-KIR2DL5 antibody, anti-NKp46 antibody, anti-NKp30 antibody, anti-NKG2D antibody, or the like. The antibody may be a monoclonal antibody, a polyclonal antibody, or the like. The selection of the objective NK cells or a population thereof may be performed by selectively removing T cells, NKT cells, hematopoietic precursor cells, and other cells.

(Effect of mixed culture)

According to the investigations of the inventors of the present invention, by culturing a population of mononuclear cells originating in a plurality of donors and including NK cells, favorable proliferation of NK cells can be attained. This is considered to originate in more mutually available licensing signals resulting from increase in variations of the combination of HLA/KIR. Favorable proliferation means that proliferation ratio of cells (cell number after culture/cell number before culture) is improved compared with that obtainable by culturing cells derived from a single donor as any one of the plurality of donors used for the mixed culture.

In the cell population obtained by the mixed culture, NK cells may account for 70% or more, preferably 80% or more, more preferably 90% or more, of the total cells.

According to the investigations of the inventors of the present invention, the cytotoxic activity of the NK cells obtained by the mixed culture of the population of mononuclear cells including NK cells of a plurality of donors may be equivalent to or higher than that of NK cells derived from a single donor. The ratio of contained high CD16 expression NK cells may also be equivalent to or higher than that of NK cells derived from a single donor. The cytotoxic activity means an ability of an objective cell (effecter cell, E) for lysing a target cell (T), unless especially indicated. The cytotoxic activity can be represented by the percentage (%) of the target cells killed by the effecter cells, and can be obtained in accordance with, for example, the following equation.

(Cell deaths observed in co-culture with effecter cells -natural cell deaths (negative control))/(maximum cell deaths (positive control) - natural cell deaths (negative control)) x 100

In general, when cytotoxic activity is measured, mixing ratio of effecter cells and target cells (E:T) and time of co-culture of effecter cells and target cells can be arbitrarily determined according to the degree of the cytotoxic activity of the effecter cell etc. as well as types and activity strengths of the cells to be used. When NK cells are used as the effecter cell, the target cell may be K562 cell, SKOV3 cell (human ovarian cancer cell strain), acute myeloid leukemia cell, or chronic myeloid leukemia cell, but the target cell is not limited to these. The effecter cells and target cells, or living cells and dead cells can be distinguished and quantified by using a reagent such as an antibody labeled with a radioactive substance, fluorescent dye, or the like.

The population of NK cells of the present invention originating in a plurality of donors does not suffer from suppression by MDSCs (myeloid-derived suppressor cells) or suffer from such suppression at extremely low level. On the basis of the investigations done so far, the inventors of the present invention confirmed that a population of NK cells originating in a single donor obtained by the aforementioned culture method does not suffer from the suppression by MDSCs. As one of the reasons for this, there can be mentioned no expression or expression at an extremely low level of receptors for the humoral factors (TGF-$\beta$, IL-10). According to the investigation of the inventors of the present invention of this time, it was found that even if cells derived from a plurality of donors are mixed, the aforementioned advantage is not impaired.

[Cell population including NK cells]

The cell population including NK cells obtained by the present invention has the following characteristics:

(1) the cell population originates in a plurality of donors,
(2) the cell population shows a cytotoxic activity of 50% or higher in co-culture of NK cells as effecter cells (E) and K562 cells as target cells (T) at a mixing ratio of 1:1 (E:T).

Instead of the aforementioned characteristic of (2), or in addition to the characteristic (2), it may have the following characteristic:
(2') it shows a cytotoxic activity of 50% or higher in co-culture of NK cells as effecter cells (E) and SKOV3 cells as target cells (T) at a mixing ratio of 3:1 (E:T).

Such a cell population may further have the following characteristics.

(3) the ratio of NK cells is 70% or higher, specifically 80% or higher, more specifically 90% or higher,
(4) it may contain both a population of CD 16 high expression NK cells and a population of CD16 low expression NK cells, and ratio of the CD16 high expression NK cells is 50% or higher.

Cytotoxic activity of NK cells can be measured and calculated by methods well known to those skilled in the art. The cytotoxic activity (%) can usually be calculated on the basis of living cell number of the target cells (T) observed after the effecter cells (E) have been allowed to act on them, in accordance with, for example, the equation: (1 — Number of living cells/number of living cells of negative control) x 100.

The cytotoxic activity observed in co-culture using the NK cells as the effecter cells (E) and the K562 cells as the target cells (T) at a mixing ratio of 1:1 (E:T) is preferably 60% or higher, more preferably 70% or higher, still more preferably 80% or higher, further preferably 90% or higher, still further preferably 95% or higher.

The cytotoxic activity observed in co-culture using the NK cells as the effecter cells (E) and the SKOV3 cells as the target cells (T) at a mixing ratio of 3:1 (E:T) is preferably 60% or higher, more preferably 70% or higher, still more preferably 80% or higher, further preferably 90% or higher, still further preferably 95% or higher.

Positivity for a marker such as CD16 may be represented with a symbol +, and negativity for such a marker may be represented with a symbol -. For example, positivity for CD16 may be represented as CD16+, and negativity for CD16 may be represented as CD16⁻. The positivity may mean both high expression and low expression. High expression characteristic may be represented as high or bright. For example, CD 16 high expression characteristic may be represented as $CD16^{high}$ or $CD16^{bright}$. Low expression characteristic may be represented as low or dim. For example, CD16 low expression characteristic may be represented as $CD16^{low}$ or $CD16^{dim}$.

The positivity, negativity, high expression characteristic, and low expression characteristic can be determined on the basis of a chart obtained by flow cytometry. Although a position observed in such a chart may change depending on voltage setting, sensitivity setting of instruments, antibody clone used, staining conditions, dye used, and so forth, those skilled in the art can appropriately draw a line so that a cell population observed as one group on the obtained chart should not be divided.

Whether a cell is positive or negative for expression of an objective marker can be determined by using a negative control using an isotype control antibody. The isotype control antibody is an antibody that does not react with specific antigen. In general, when an experiment is performed by using an antibody, background may be generated by nonspecific bindings with proteins other than the target, and binding with the Fc receptors on cell surfaces. By comparison with a system using an antibody that serves as the negative control, specificity of the reaction of the primary antibody to the target antigen can be clarified. Further, the influence of the background is eliminated, and strength of a signal can be accurately interpreted.

Degree of expression of a target marker (low expression or high expression) can be determined by comparison with data for control cells measured under the same conditions. Examples of the control cell include NK cells obtained from peripheral blood and not substantially cultured such as the primary NK cells mentioned in the section of Examples of this description.

For example, as for degree of expression of CD16 in a certain population of NK cells, CD 16 expression amount in the cell population can be compared with CD 16 expression amount of a population of NK cells obtained from peripheral blood and not substantially cultured (control) by using flow cytometry, and when expression equivalent to that of the control is observed, it can be determined to have high expression characteristic, or when expression is lower than that of the control cells, it can be determined to have low expression characteristic. The NK cells of the control are known to have high CD 16 expression characteristic.

[Use as drug]

The present invention also provides a pharmaceutical composition for cell therapy, which comprises the aforementioned cell population including NK cells as an active ingredient. The cell therapy means a method for treating a disease or condition of an object by administering cells treated out of the body, and includes immunocyte therapy.

The pharmaceutical composition contains, in addition to the cell population as the active ingredient, a solution that can suspend the NK cells, for example, physiological saline, phosphate-buffered saline (PBS), or the like. The pharmaceutical composition or solution may also contain pharmaceutically acceptable additives. The pharmaceutical composition can be administered, for example, intravenously, intraarterially, subcutaneously, or intraperitoneally. The cell therapy based on the pharmaceutical composition can be performed independently, or in combination with a surgical therapy, chemotherapy, radiotherapy, or the like.

Applications of cell populations including NK cells to cancer therapy or infectious disease treatment are expected (Dahlberg C.M. et al., Natural Killer Cell-Based Therapies Targeting Cancer: Possible Strategies to Gain and Sustain Anti-Tumor Activity, Front. Immunol., 2015;30, https://doi.org/10.3389/fimmu.2015.00605; and Schmidt S. et al, Natural killer cells as a therapeutic tool for infectious diseases - current status and future perspectives, Oncotarget, 2018;9(29):20891-20907). The pharmaceutical composition of the present invention can be used for treating such cancers or infectious diseases. More specifically, they include mouth cancer, gallbladder cancer, bile duct cancer, lung cancer, liver cancer, colon cancer, kidney cancer, vesical cancer, leukemia; infectious diseases caused by viruses and bacteria, and so forth, but not limited to these. In a cell therapy using the pharmaceutical composition of the present invention, NK cells may be administered, for example, intravenously, intraarterially, subcutaneously, intraperitoneally, or the like. The cell therapy may be performed independently, or in combination with a surgical therapy, chemotherapy, radiotherapy, antibody drug, or the like.

Many of antibody drugs are supposed to exhibit an antitumor effect based on the ADCC activity after intravenous administration. However, when the ADCC activity is exhibited, monocytes, macrophages, and neutrophils are also recruited in addition to the NK cells. The effecters other than the NK cells show the ADCC activity without distinguishing cancer cells from normal cells, and it is considered that this also leads to expression of side reactions. According to the present invention, the NK cells and antibodies may be mixed before administration, so that the NK cells are equipped with antibodies beforehand. The effecter is thereby limited to the NK cells, thus amount of the antibody to be administered can be reduced, and it is also considered to be very effective for reducing the side reactions. That is, the pharmaceutical composition of the present invention may be prepared through the step of mixing the cell population of NK cells and antibodies and then removing antibodies not binding with NK cells. That is, the pharmaceutical composition of the present invention according to one of the preferred embodiments contains NK cells and antibodies, wherein the antibodies bind to the NK cells, and antibodies not binding to the NK cells are not substantially contained (refer to Patent document 3 mentioned above).

The pharmaceutical composition of the present invention is preferably produced under conditions conforming to the rules for production control and quality control of drugs and quasi-drugs (Good Manufacturing Practice, GMP) and the criteria for production control and quality control of products for regenerative medicine etc. (Good Gene, Cellular, and Tissue-based Products Manufacturing Practice, GCTP).

The examples of the present invention explained below are mentioned for only the purpose of exemplification, and do not limit the technical scope of the present invention. The technical scope of the present invention is limited only by the descriptions of the claims. The present invention can be modified, for example, essential elements of the present invention may be added, deleted, or replaced, on condition that such change including addition, deletion and replacement of essential elements does not depart from the spirit of the present invention.

Examples

[Example 1: Mixed culture of NK cells obtained from fresh peripheral blood 1]

Blood was collected from healthy volunteers, and peripheral blood mononuclear cells were isolated by density gradient centrifugation using Ficoll (GE Healthcare, 17144002). The isolated peripheral blood mononuclear cells of a plurality of persons were mixed at substantially equal ratios, CD3 beads[*1] were added and suspended, incubation was performed at 4°C for 15 minutes, then 1 mL of a separation buffer[*2] was added, the cells were sufficiently suspended therein, and centrifugation was performed at 300 x g for 10 minutes. The supernatant was removed, the cells were suspended in 0.5 mL of the separation buffer, the suspension was applied to an LD column (Miltenyi Biotec, 130-042-901) moistened beforehand by adding 2 mL of the separation buffer, and the eluate eluted from the LD column was collected. The separation buffer in a volume of 1 mL was further applied to the LD column, and the eluate was collected. Then, the column was washed with 1 mL of the separation buffer, the number of the cells in the collected liquid was counted, and the total cell number was calculated. Centrifugation was performed at 500 x g for 5 minutes, the supernatant was removed, and then the cells were suspended in the KBM501 medium[*3] at a density of 5 x $10^5$ cells/mL.

A part of the cells were collected for flow cytometry, and the remaining cells were cultured. The culture was performed by using a 6-well plate (Thermo Fisher Scientific, 140675), T-75 flask (Thermo Fisher Scientific, 156499), or 500-cm$^2$ culture bag (Nipro) in a $CO_2$ incubator (37°C, 5% $CO_2$). A part of the culture liquid was taken on the days 5 and 9 of the culture, the cell number was counted, and the KBM501 medium was added to the culture liquid on the day 9 to attain a final liquid volume of 6 mL per well in the case of the 6-well plate, or 50 mL per flask in the case of the T-75 flask. The cells were collected on the day 14, the cell number was counted, and by using a part of the cells, cell surface antigens were measured with a flow cytometer.

*1: CliniMACS CD3, Miltenyi Biotec, 130-017-601 (5 μL per 1 × $10^7$ cells)

*2: PBS (Wako Pure Chemical Industries industry, 045-29795) containing 0.5% human type AB serum (Cosmobio, 12181301, inactivated at 56°C for 30 minutes) and 2 mM EDTA (Thermo Fisher Scientific, 15575-020)

*3: KBM 501 (Kohjin-bio, 16025015) containing 5% human type AB serum (Cosmobio, 12181301, inactivated at 56°C for 30 minutes)

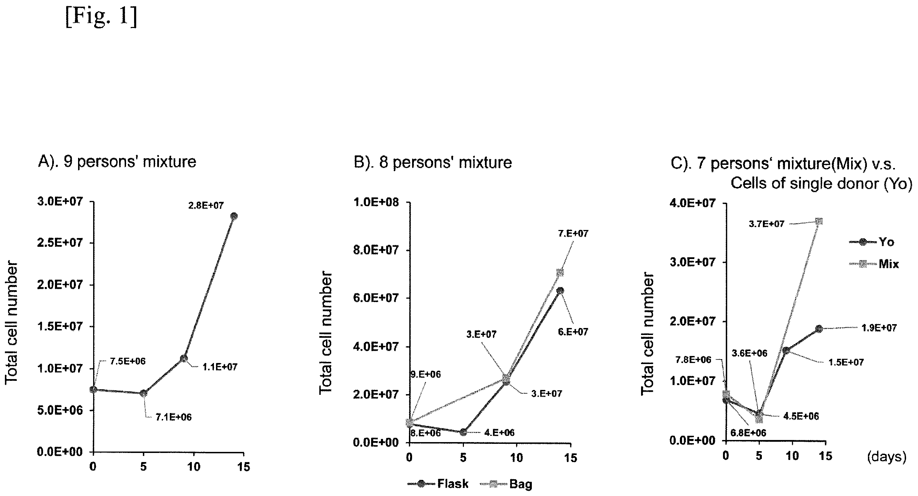

The results are shown in Fig. 1. The cells favorably proliferated in all of the 9 persons' mixture (Fig. 1, A), 8 persons' mixture (Fig. 1, B), and 7 persons' mixture (Fig. 1, C). When purities were confirmed, 90% or more of the cells were CD3[-] and CD56[+] NK cells in all the mixed cultures. As for the 8 persons' mixture, the mixed culture was performed in the T-75 flask and 500-cm$^2$ culture bag, and the cell proliferation was favorable in the both culture vessels. As for the 9 persons' mixture, 7 persons' mixture, and cells of a single donor (one of the 7 persons), the culture was performed by using the 6-well plate. In the culture using the 500-cm$^2$ culture bag, an operation of turning the bag over was performed as described in Example 4 mentioned later.

As comparison of the cell proliferations in the culture of 7 persons' mixture and culture of cells derived from a single donor, whereas 6.8 x $10^6$ of the cells proliferated to 1.9 × $10^7$ cells (about 2.8 times) in the culture of cells derived from a single donor, 7.8 × $10^6$ cells proliferated to 3.7 × $10^7$ cells (about 4.7 times) in the mixed culture, and the mixed culture showed more favorable cell proliferation (Fig. 1, C).

[Example 2: Mixed culture of NK cells obtained from fresh peripheral blood 2]

The data obtained from the culture of the cells derived from a single (individual) donor (n = 17) and mixed cultures of the cells of 4 or more persons (n = 10) performed in T-75 flasks in the same manner as described in Example 1 were combined, and statistical analysis was performed for the obtained cell proliferation rates for the cell numbers of NK cells. The Wilcoxon rank sum test was performed by using JMP Pro13 as the analysis software.

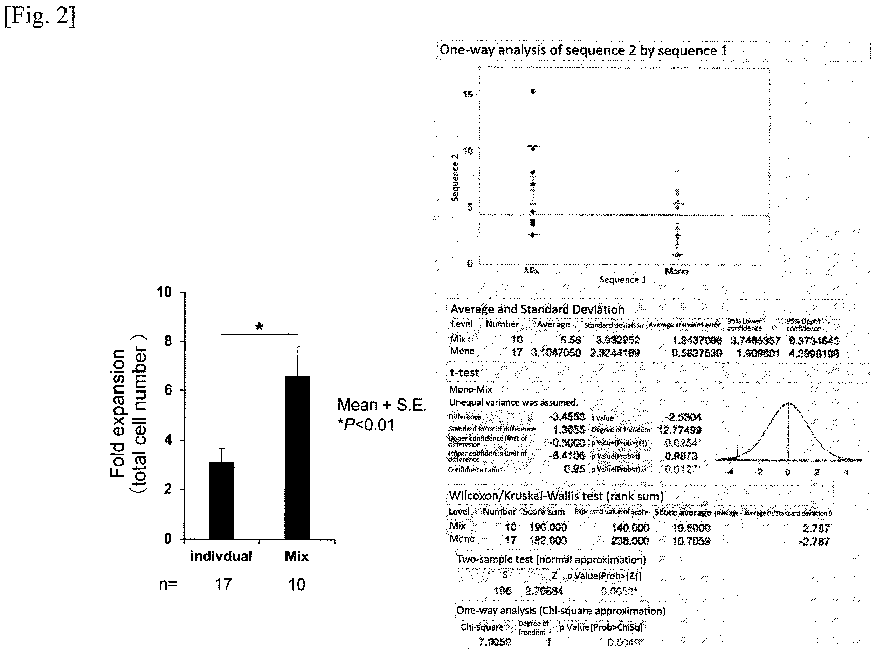

The results are shown in Fig. 2. Whereas the proliferation (expansion) ratio (total cell number after proliferation/total cell number before proliferation) in the culture of the cells derived from the single (individual) donor was 3.10 $\pm$ 0.56, the proliferation ratio in the mixed culture was 6.56 $\pm$ 1.24, and thus the mixed culture provided a statistically significantly higher proliferation ratio ($p < 0.01$).

[Example 3: Tumor cell cytotoxic activity test]

«Preparation of NK cells»

Mixed cultured NK cells of four persons and cultured NK cells of a single (individual) donor (one person of the four persons) obtained from healthy volunteers by the method described in Example 1 were collected, washed, and then suspended in the RPMI 1640 medium (Wako Pure Chemical Industries, 189-02025) containing 10% FBS (Nichirei Bioscience, 171012-500ML), 100 units of penicillin, and 100 $\mu$g/mL of streptomycin (Nacalai Tesque, 26253-84) (henceforth referred to as 10% FBS/RPMI 1640), and the density was adjusted to $1 \times 10^6$ cells/mL with the same medium.

«Preparation of SKOV3»

SKOV3 cells (human ovarian cancer cell strain) were prepared at a density of $1 \times 10^6$ cells/mL in serum component-free RPMI 1640 medium (Wako Pure Chemical Industries industry, 189-02025). The prepared SKOV3 cells were stained by using PKH26 Red Fluorescent Cell Linker Kit (Sigma, PKH26GL-1KT), and finally prepared at densities of $2 \times 10^6$ cells/mL and $2 \times 10^5$ cells/mL with 10% FBS/RPMI 1640.

«Preparation of MDSCs (myeloid-derived suppressor cells)»

Peripheral blood mononuclear cells were isolated from a healthy volunteer (different from the donors of NK cells) by using Ficoll, and cultured in 10% FBS/RPMI 1640 containing 10 ng/mL of IL-6 (PEPROTECH, 200-06-5UG) and 10 ng/mL of GM-CSF (CellGenix, 1012-050) for 7 to 10 days to induce MDSCs. MDSCs were suspended in the serum component-free RPMI 1640 medium (Wako Pure Chemical Industries, 189-02025), and stained by using PKH Green Fluorescent Cell Linker Kit (Sigma, MINI67-1KT), and the suspension was finally adjusted to a density of $8 \times 10^5$ cells/mL with 10% FBS/RPMI 1640.

«Cytotoxic activity test»

A group of the mixed cultured NK cells derived from a plurality of donors and SKOV3 cells, a group of cultured NK cells derived from a single donor and SKOV3 cells, groups corresponding to the foregoing 2 groups, but further containing MDSCs, 4 groups in total, as well as a group of only SKOV3 cells as a negative control were prepared.

The NK cells and SKOV3 cells were inoculated on a 96-well plate (IWAKI, 4870-800SP) at a cell number ratio of 3:1, mixed, and allowed to react at 37°C under 5% $CO_2$ for 4 hours. As for the groups containing MDSCs, NK cells, SKOV3 cells and MDSCs were used at a cell number ratio of 5:1:4. The NK cells and MDSCs were first mixed on a 96-well plate, and allowed to react at 37°C under 5% $CO_2$ for 12 to 18 hours. Then, centrifugation (500 x g, 5 minutes) was performed, the supernatant was removed, then the SKOV3 cells were added and mixed, and the reaction was allowed at 37°C under 5% $CO_2$ for 4 hours. After the reaction, the centrifugation (500 x g, 5 minutes) was performed, the supernatant was removed, the Zombie solution (Biolegend, 423105) diluted with PBS was added and mixed, and the mixture was incubated at room temperature for 30 minutes in a dark place. The mixture was centrifuged, the supernatant was removed, the cells were suspended in PBS, and AccuCheck Counting Beads (Thermo Fisher Scientific, PCB100) were added. Measurement was performed by using a flow cytometer (BD LSR Fortessa, BD Bioscience), and the results were analyzed with FlowJo software (FLOWJO, LLC) to calculate the cytotoxic activity ratio (%Lysis)[*4].

$$*4: \text{Cytotoxic activity ratio} = (1 - (\text{SKOV3 living cell count})/(\text{SKOV3 living cell count of negative control})) \times 100$$

Living cell count: Actually measured SKOV3 cell count x number of beads in added beads suspension/actually measured number of beads

Actually measured SKOV3 cell count: FSC/SSC gated (debris exclusion), and PKH26[+] gated after doublet exclusion

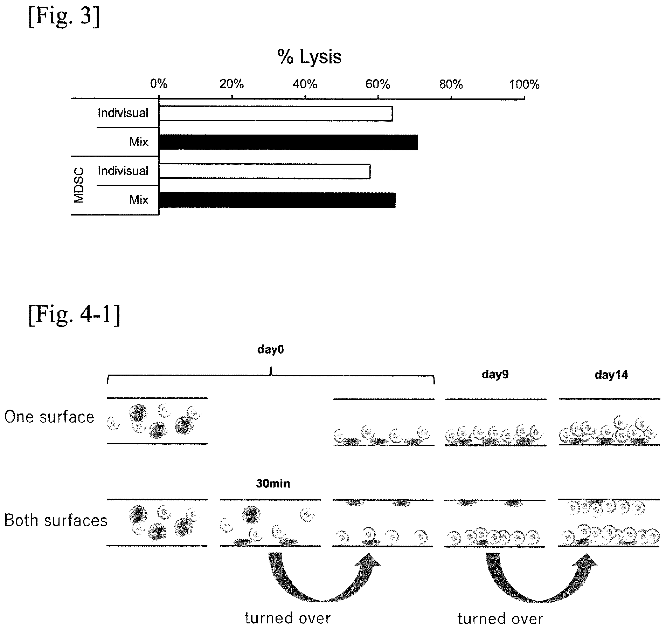

The results are shown in Fig. 3. The mixed cultured NK cells derived from of a plurality of donors showed cytotoxic activities equally or higher than those of the NK cells derived from a single (individual) donor.

[Example 4: Mixed culture of NK cells prepared from frozen apheresis blood]

Frozen apheresis bloods of two donors (HemaCare, PB001CLP) were thawed, mixed, diluted with HBSS(-) solution (Nacalai Tesque, 17461-05), and washing with PBS/EDTA solution (Miltenyi Biotec, 130-021-201) and concentration were performed by using an automatic closed system cell processing unit, Lovo Cell Processing System (FRESENIUS KABI). Then, by using CliniMACS Prodigy TS 310 (Miltenyi Biotec, 130-097-183), CliniMACS CD3 MicroBeads (Miltenyi Biotec, 130-017-601), and CliniMACS CD34 Reagent (Miltenyi Biotec, 130-017-501), CD3-positive cells and CD34-positive cells were removed from the concentrated cell suspension, and washing and elution were performed. Cell number in the eluate was determined to calculate the total cell number. The suspension was centrifuged at 500 x g for 5 minutes, the supernatant was removed, and then the cells were suspended in the KBM501 medium at a density of $1 \times 10^6$ cells/mL. The culture was performed by using the T-75 flask (Thermo Fisher Scientific, 156499) or 500-cm$^2$ culture bag (Nipro) in a $CO_2$ incubator (37°C, 5% $CO_2$). In the case of the culture using the bag, an operation of turning the bag over after 30 minutes from the start of the culture, and turning the bag over again on the day 9 of the culture was performed (refer to Fig. 4-1). On the day 9 of the culture, a part of the culture liquid was collected, cell number was counted, and the KBM 501 medium was added to obtain a final volume of 50 mL per flask in the case of using the T-75 flask, or 500 mL per bag in the case of using the culture bag. On the day 14, the cells were collected, the total cell number was counted, and cell surface antigens were measured for a part of the cells by using a flow cytometer.

The collected cells were stained by using antibodies as follows. Staining was performed by using Alexa Fluor® 700-labelled anti-human CD56 antibody (Biolegend, 318316), PerCP/Cy5.5-labelled anti-human CD3 antibody (Biolegend, 300430), and PE-Cy7-labelled anti-human CD16 antibody (Biolegend, 302016) at a concentration of 1 µg/mL at 4°C for 30 minutes, and then the centrifugation (500 x g, 5 minutes, 4°C) was performed. The supernatant was removed, the cells were suspended in PBS(-) (Wako Pure Chemical Industries), then measurement was performed by using a flow cytometer (BD LSRFortessa, BD Bioscience), and the obtained data were analyzed by using FlowJo software (FLOWJO, LLC).

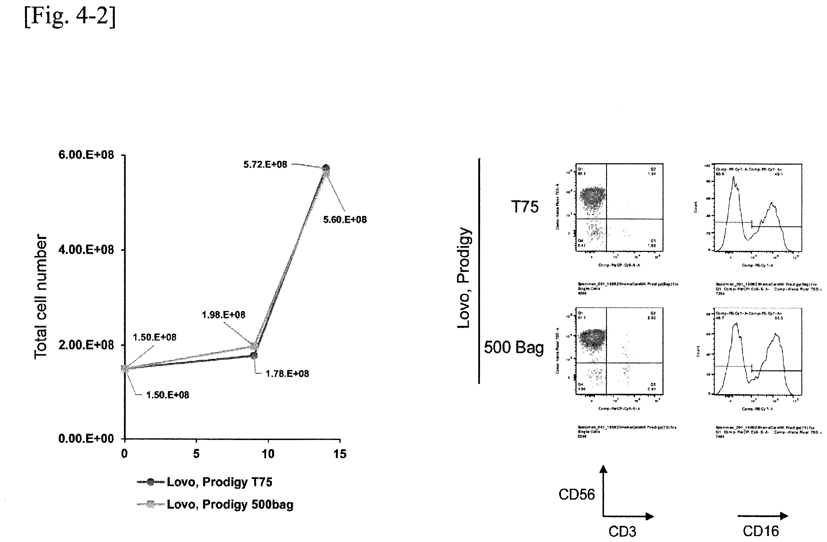

The results are shown in Fig. 4-2. Also when the NK cells obtained from the frozen apheresis blood were used, the cells were fully proliferated by the mixed culture in 14 days. More precisely, $1.50 \times 10^8$ cells proliferated to $5.72 \times 10^8$ cells (about 3.81 times) in the case of the T-75 flask culture, and $1.50 \times 10^8$ cells proliferated to $5.60 \times 10^8$ cells (about 3.73 times) in the case of the 500-cm$^2$ culture bag culture (Fig. 4-2, left).

Purity of the NK cells in the obtained population of the cultured NK cells was 90.5% in the case of the T-75 flask culture, or 91.7% in the case of 500-cm$^2$ culture bag culture. As for expression of CD16, the population of the mixed cultured NK cells showed bimodality, like the population of the cultured NK cells derived form a single donor (refer to Japanese Patent Unexamined Publication (KOKAI) No. 2018-193303). More precisely, the ratios of CD16 low expression and CD16 high expression were 50.9% and 49.1%, respectively, in the case of the T-75 flask culture, or 46.7% and 53.3%, respectively, in the case of the 500-cm$^2$ culture bag culture (Fig. 4-2, right).

[Example 5: Monocytes/NK cells swapping experiment]

«Preparation of primary NK cells and monocytes»

Blood was collected from two of healthy volunteers (referred to as donors 1 and 2), and peripheral blood mononuclear cells were isolated by density gradient centrifugation using Ficoll. From the isolated peripheral blood mononuclear cells, primary NK cells were isolated by using EasySep™ Human NK Cell Enrichment Kit (STEMCELL, 19055), monocytes were separated by using EasySep™ Human Monocyte Enrichment Kit without CD16 Depletion (STEMCELL, 19058), and the cell numbers of them were counted. The primary NK cells were suspended in the KBM 501 medium at a density of $1 \times 10^5$ cells/mL, and the monocytes were suspended in the same medium at a density of $3 \times 10^5$ cells/mL.

«Swapping culture»

Total 4 groups consisting of combinations of the primary NK cells of the donor 1 and the monocytes of the donor 1, the primary NK cells of the donor 1 and the monocytes of the donor 2, the primary NK cells of the donor 2 and the monocytes of the donor 2, and the primary NK cells of the donor 2 and the monocytes of the donor 1, respectively, were prepared by mixing each primary NK cells and monocytes at a cell number ratio of 1:3, and culture was started on 6-well plates (Thermo Fisher Scientific, 140675) (37°C, 5% $CO_2$). On the day 9 of the culture, the KBM501 medium was added, and the cells were collected on the day 14 as in the method described in Example 1. For the obtained cells, cell surface antigens and cytotoxic activity ratio with respect to K562 (human chronic myeloid leukemia cell strain) were measured.

As for the measurement of cell surface antigens, the collected cells were stained by using antibodies, and analyzed as follows.

Staining was performed by using Alexa Fluor® 700-labelled anti-human CD56 antibody (Biolegend, 318316), APC/Cy7-labelled anti-human CD3 antibody (Biolegend, 300426), FITC-labelled anti-human KIR2DL1 antibody (Miltenyi Biotec, 130-103-966), and PerCP/Cy5.5-labelled anti-human KIR3DL1 antibody (Biolegend, 312718) at a concentration of 1 $\mu$g/mL at 4°C for 30 minutes, and then centrifugation was performed (500 x g, 5 minutes, 4°C). The supernatant was removed, the cells were suspended in PBS(-) (Wako Pure Chemical Industries), measurement was performed by using a flow cytometer (BD LSRFortessa, BD Bioscience), and the obtained data were analyzed by using FlowJo software (FLOWJO, LLC).

For the measurement of cytotoxic activity, 4 groups in which respective cultured NK cells and K562 cells obtained by combining the primary NK cells and monocytes of the two donors were reacted, a group using only the K562 cells as a negative control, and a group using the K562 cells fixed with 10% formalin as a positive control were prepared.

«Preparation of K562»

The K562 cells (human chronic myeloid leukemia cell strain) were suspended in the serum component-free RPMI 1640 medium (Wako Pure Chemical Industries, 189-02025) at a density of $1 \times 10^6$ cells/mL. The prepared K562 cells were stained by using PKH26 Red Fluorescent Cell Linker Kit (Sigma, PKH26GL-1KT), and the cell density was finally adjusted to $2 \times 10^6$ cells/mL with 10% FBS/RPMI 1640.

«Preparation of cultured NK cells»

The respective cultured NK cells of the 4 groups obtained by culturing combinations of the primary NK cells and monocytes of the 2 donors obtained by the aforementioned method were collected, washed, and suspended in 10% FBS/RPMI 1640, and the cell density was adjusted to $1 \times 10^6$ cells/mL with the same medium.

«Cytotoxic activity test»

The NK cells and K562 cells were added to a 96-well plate (IWAKI, 4870-800SP) at a cell number ratio of 1:1, mixed, and allowed to react at 37°C under 5% $CO_2$ for 2 hours. After the reaction, centrifugation (500 x g, 5 minutes) was performed, the supernatant was removed, then 7-AAD solution (Beckman Coulter, A07704) diluted with PBS was added to the cells to suspend them, and the suspension was incubated at room temperature for 20 minutes. Measurement was performed by using a flow cytometer (BD LSRFortessa, BD Bioscience), the obtained data were analyzed by using FlowJo software (FLOWJO, LLC), and cytotoxic activity ratio (%Lysis) was calculated[*5] .

*5: Cytotoxic activity ratio = (Dead K562 cell ratio — dead cell ratio of negative control)/(dead cell ratio of positive control - dead cell ratio of negative control) x 100

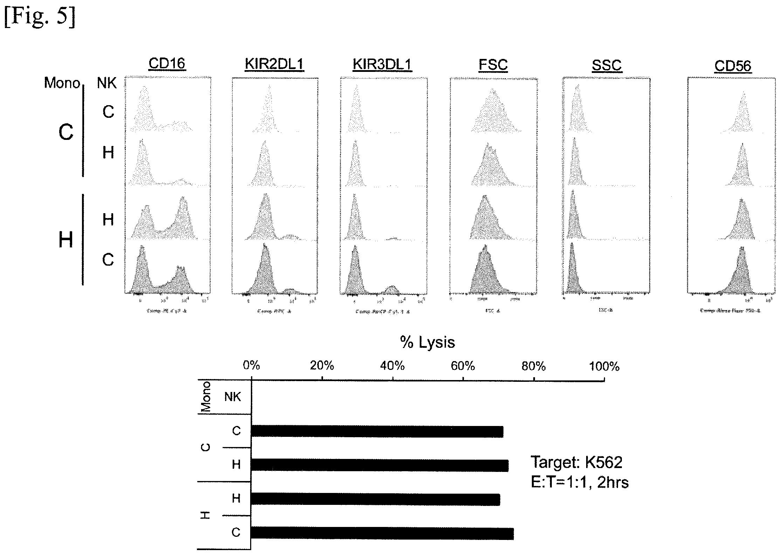

The donor typing information is shown in the following table, and the results are shown for in Fig. 5.

[Table 1]

|  | Monocyte | Bw4 | C1 | C2 | HLA-F |
|---|---|---|---|---|---|
|  | Donor 1 | - | + | - | + |
|  | Donor 2 | + | + | - | + |
| Negative signal | NK cell | KIR3DL1 | KIR2DL2 | KIR2DL1 |  |
|  | Donor 1 | + | -/+ | + |  |
|  | Donor 2 | + | -/+ | + |  |
| Positive signal | NK cell | KIR3DS1 | KIR2DS2 | KIR2DS1 | KIR3DS1 |
|  | Donor 1 | - | - | - | - |
|  | Donor 2 | + | - | + | + |

Under the conditions that one or both of signals from KIR3DL1 and KIR3DS 1 are available, populations of CD16[high] increase, and therefore higher ADCC activity can be expected.

**Claims**

1.  A method for producing a cell population including NK cells, which comprises:

    preparing a cell population of mononuclear cells originating in a plurality of donors and including NK cells
    incubating the prepared population of mononuclear cells under conditions effective for treating NK cells.

2.  The production method according to claim 1, wherein the step of preparing a population of mononuclear cells comprises the step of removing CD3-positive cells.

3.  The production method according to claim 1 or 2, wherein the step of preparing a population of mononuclear cells comprises the step of removing CD34-positive cells.

4.  The production method according to any one of claims 1 to 3, wherein the step of preparing a population of mononuclear cells comprises the step of obtaining a population of mononuclear cells from peripheral blood collected from a plurality of donors.

5.  The production method according to any one of claims 1 to 4, wherein the step of preparing a population of mononuclear cells comprises the step of obtaining a population of mononuclear cells from apheresis blood collected from a plurality of donors.

6.  The production method according to any one of claims 1 to 3, wherein the step of preparing a population of mononuclear cells consists of preparing a population of mononuclear cells derived from any one selected from the group consisting of embryonic stem (ES) cells, induced pluripotent stem (iPS) cells, and adult stem cells originating in a plurality of donors.

7.  The production method according to any one of claims 1 to 6, wherein the plurality of donors include one donor and another donor having a genotype of at least one of HLA or KIR different from that of the foregoing one donor.

8.  A cell population including NK cells, which has the following characteristics:

    (1) the cell population originates in a plurality of donors,
    (2) the cell population shows a cytotoxic activity of 50% or higher in co-culture of NK cells as effecter cells (E) and K562 cells as target cells (T) at a mixing ratio of 1:1 (E:T).

9.  A pharmaceutical composition for cell therapy, which comprises a cell population produced by the production method according to any one of claims 1 to 7.

10. A pharmaceutical composition for cell therapy, which comprises the cell population according to claim 8.

11. The pharmaceutical composition according to claim 9 or 10, for use in treating an infectious disease and/or cancer.

[Fig. 1]

[Fig. 2]

Mean + S.E.
*P<0.01

n=    17    10

[Fig. 3]

% Lysis

[Fig. 4-1]

[Fig. 4-2]

[Fig. 5]

## INTERNATIONAL SEARCH REPORT

| International application No. |
|---|
| PCT/IB2020/052499 |

**A.    CLASSIFICATION OF SUBJECT MATTER**
A61P  31/04(2006.01)i;  A61P  35/00(2006.01)i;  C12N  5/0783(2010.01)i;  A61K
35/17(2015.01)i
FI: C12N5/0783; A61P35/00; A61P31/04; A61K35/17 Z

According to International Patent Classification (IPC) or to both national classification and IPC

**B.    FIELDS SEARCHED**

Minimum documentation searched (classification system followed by classification symbols)
A61P31/04; A61P35/00; C12N5/0783; A61K35/17

| Documentation searched other than minimum documentation to the extent that such documents are included in the fields searched | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
CAplus/MEDLINE/EMBASE/BIOSIS/WPIDS (STN) JSTPlus/JMEDPlus/JST7580
(JDreamIII)

**C.    DOCUMENTS CONSIDERED TO BE RELEVANT**

| Category* | Citation of document, with indication, where appropriate, of the relevant passages | Relevant to claim No. |
|---|---|---|
| X<br>Y | JP 2017-508479 A (EMERCELL SAS) 30.03.2017 (2017-03-30) paragraphs [0011], [0014], [0076], [0084], [0095], [0108], examples 4, 5 | 1,2,7–11<br>3–6 |
| X<br>Y | SUGITA, Sunao et al., "Natural Killer Cell Inhibition by HLA-E Molecules on Induced Pluripotent Stem Cell-Derived Retinal Pigment Epithelial Cells", Investigative Ophthalmology & Visual Science, April 2018, vol. 59, no. 5, pp. 1719-1731, p. 1721, column "RESULTS" | 1,2,4,6–11<br>3–6 |

☒    Further documents are listed in the continuation of Box C.          ☒    See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 01 June 2020 (01.06.2020) | 09 June 2020 (09.06.2020) |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

20

**INTERNATIONAL SEARCH REPORT**

International application No.

PCT/IB2020/052499

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | 増田亜希子, II．リンパ系腫瘍の基本事項 4．細胞表面形質，日本内科学会雑誌，July 2011, vol. 100, no. 7, pp. 1807-1816, table 1, (MASUDA, Akiko, "II. Basic Knowledge of Lymphoid Malignancies, 4. Cell Surface Markers", Nihon Naika Gakkai Zasshi) | 3<br>1,2,4-11 |
| Y<br>A | JP 2014-80431 A (KYUSHU UNIVERSITY) 08.05.2014 (2014-05-08) paragraph [0022] | 4-6<br>1-3,7-11 |
| Y<br>A | WO 2014/112491 A1 (ABE, Hiroyuki) 24.07.2014 (2014-07-24) paragraphs [0014], [0015] | 4-6<br>1-3,7-11 |
| Y<br>A | JP 2016-187341 A (GAMIDA-CELL LTD.) 04.11.2016 (2016-11-04) paragraph [0128] | 4-6<br>1-3,7-11 |

Form PCT/ISA/210 (continuation of second sheet) (January 2015)

**INTERNATIONAL SEARCH REPORT**
Information on patent family members

International application No.

PCT/IB2020/052499

| Patent Documents referred in the Report | Publication Date | Patent Family | Publication Date |
|---|---|---|---|
| JP 2017-508479 A | 30 Mar. 2017 | WO 2015/132415 A1<br>p. 2, lines 27-30, p. 3, lines 10-12, p. 16, lines 16-20, p. 17, lines 24-32, examples 4, 5<br>EP 3114215 A1<br>US 2017/0073637 A1 | |
| JP 2014-80431 A | 08 May 2014 | US 2014/0120072 A1<br>paragraph [0020]<br>EP 2725100 A1<br>WO 2012/176796 A1 | |
| WO 2014/112491 A1 | 24 Jul. 2014 | US 2016/0024471 A1<br>paragraphs [0019], [0020]<br>EP 2947144 A1<br>WO 2014/112491 A1 | |
| JP 2016-187341 A | 04 Nov. 2016 | WO 2011/080740 A1<br>p. 35<br>US 2013/0011376 A1<br>EP 2519239 A1<br>EP 3184109 A1 | |

Form PCT/ISA/210 (patent family annex) (January 2015)

**EP 3 915 640 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2013027385 A
- JP 5572863 B
- JP 5989016 B
- JP 2014226079 A
- JP 5511039 B
- JP 6164650 B
- JP 2018193303 A
- JP 2018059624 A

### Non-patent literature cited in the description

- **MINGUS J.J. ROSE et al.** Killer Ig-Like Receptor Ligand Mismatch Directs NK Cell Expansion In Vitro. *The Journal of Immunology,* 2009, vol. 183, 4502-4508
- **HIROSHI ICHISE et al.** NK Cell Alloreactivity against KIR-Ligand-Mismatched HLA-Haploidentical Tissue Derived from HLA Haplotype-Homozygous iPSCs. *Stem Cell Reports,* 2017, vol. 9, 853-867
- **DOMOGALA A et al.** Natural killer cell immunotherapy: from bench to bedside. *Frontiers in Immunology,* 2015, vol. 6
- **ZENG J et al.** Generation of ''Off-the-Shelf'' Natural Killer Cells from Peripheral Blood Cell-Derived Induced Pluripotent Stem Cells. *Stem Cell Reports,* 2017, vol. 9, 1796-1812
- **SAITO S et al.** Ex vivo generation of highly purified and activated natural killer cells from human peripheral blood. *Hum. Gene Ther. Methods,* 2013, vol. 24 (4), 241-252
- **DAHLBERG C.M. et al.** Natural Killer Cell-Based Therapies Targeting Cancer: Possible Strategies to Gain and Sustain Anti-Tumor Activity. *Front. Immunol.,* 2015, vol. 30
- **SCHMIDT S et al.** Natural killer cells as a therapeutic tool for infectious diseases - current status and future perspectives. *Oncotarget,* 2018, vol. 9 (29), 20891-20907